# EUROPEAN PATENT SPECIFICATION

(11) **EP 0 364 461 B1**
(45) Date of publication and mention of the grant of the patent: **14.09.1994**
(21) Application number: 88903973.1
(22) Date of filing: 13.04.1988
(51) Int. Cl.: C12M 1/26, C12Q 1/24, B01F 13/08

(54) **A DEVICE AND A METHOD FOR SPREADING MICROORGANISMS**
ANORDNUNG UND VERFAHREN ZUM AUSSPREIZEN VON MIKROORGANISMEN
DISPOSITIF ET PROCEDE PERMETTANT D'ETALER DES MICRO-ORGANISMES

(30) Priority: 16.04.1987 SE 8701598
(43) Date of publication of application: 25.04.1990
(73) Proprietor: CONWAY, Patricia, S-414 76 Göteborg (SE); WRANGSTADH, Michael, S-413 16 Göteborg (SE)
(72) Inventor: CONWAY, Patricia, S-414 76 Göteborg (SE); WRANGSTADH, Michael, S-413 16 Göteborg (SE)
(74) Representative: Lautmann, Kurt O.
(86) International application number: SE8800188
(87) International publication number: WO8808024

(56) References cited:
- GB-A- 1 164 949
- US-A- 3 892 632

## Description

The two most frequently used microbiological methods are the enumeration of microorganisms and the isolation of pure cultures from contaminated samples. Viable cells are enumerated by initially serially diluting in 10-fold steps and subsequently spreading an aliquot on an agar plate for determination of numbers of colony forming units. A spreader device of glass or plastic, is generally used to inoculate the plate. To avoid uneven distribution of colonies on the agar plate it is important that the plate be rotated during the spreading operation and that the plate be spread until the inoculum has been absorbed into the agar. Should this precaution not be observed, bacteria can continue to multiply in the fluid film and give rise to up to 10-fold higher counts. In most laboratories, this operation is completely manual, or at best, a manually operated turn table is used to rotate the plate.

Pure cultures are obtained by serially diluting the inocula on agar plates to permit growth of individual colonies. Colony isolation is generally performed using wire, platinum or disposable polystyrene loops. Samples are sucessfully diluted on agar plates by the process known as a streak dilution method which involves numerous streak on the plate to sufficiently dilute the inoculum. A common difficulty with this methodology is to dilute sufficiently so as to yield single colonies without excess dilution, thereby obtaining very few individual colonies.

Attempts to solve both these time consuming and tedious spreading operations by means of automated spreading devices for the enumeration of micro-organisms (1) and for colony isolation (2) have been reported. In addition, a rotating table on which the inoculated plate is placed and the inoculum manually spread by the operator with a bacteriological loop in a spiral mode has been described (3).It seems that although they are efficient and give reproducible results, most automated devices tend to be complicated, cumbersome and expensive to manufacture. In order to reduce the tedium associated with manual plate spreading for enumeration and isolation without excessive costs, a device was constructed in our laboratory which utilizes the principles reported by Frazer-Williams and Banbury 3. The device, the rotaplater, uses the stirring capacity of a magnetic stirrer to rotate the agar plate. The device will hold and rotate the agar plate during the spreading and colony isolation procedure.

The rotaplater will be further described in the following drawings, where
- Fig 1: shows the upper part of the rotaplater, and
- Fig 2: shows the lower part of the rotaplater, and
- Fig 3: shows both parts put together in one unit.

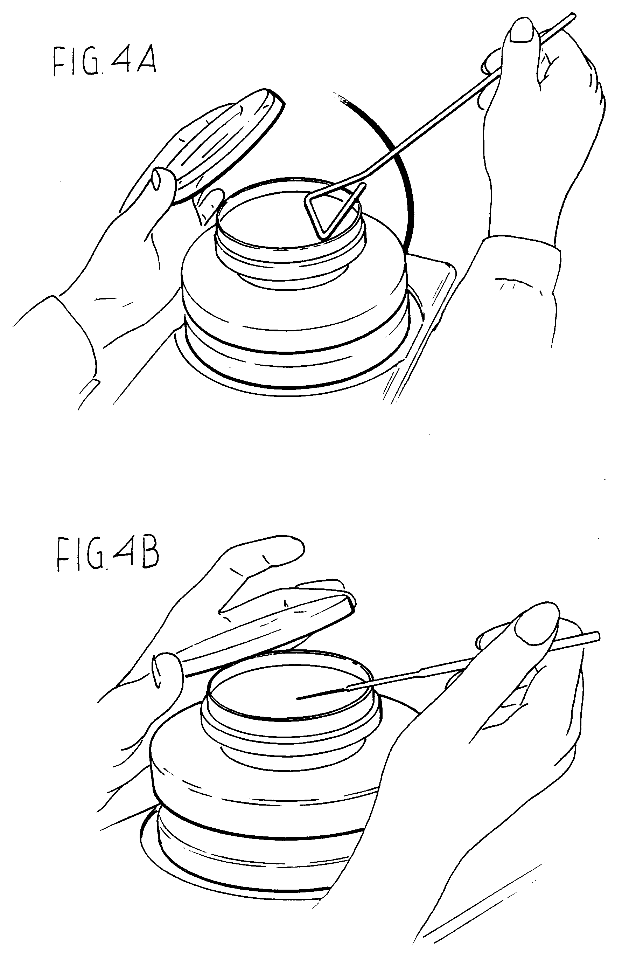

Furthermore, we refer to five drawings, Fig. 4A-B and Fig 5A-C.

### Description

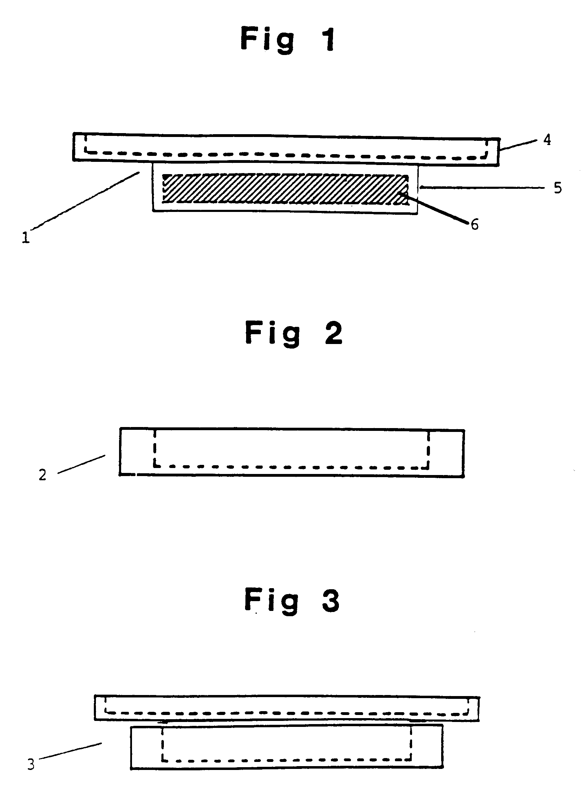

The rotaplater consists of two parts, an upper part 1 and a lower part 2. The two parts put together in one unit 3 is shown in Fig. 3. This is the unit that is placed on a magnetic stirrer.

The upper part 1 consists of a plate 4 and below this an attached part 5 containing the magnet 6. The upper part 1 can be made in one piece in which the magnet 6 is inserted.

The lower part 2 is stabilised on the magnetic stirrer by double sided tape, alternatively, it can be made in a heavier material, which will exclude the use of the double sided tape. By the use of the magnet 6 mounted in the lower part 5, the upper part 1 rotates in the lower part 2.

The rotaplater can be made in plastic. A suitable material is DELRIN®. The rotating part 5 and the lower part 2 can preferably be made out of TEFLON® or coated with TEFLON® in order to reduce friction. The choice of material can be varied if due precaution is taken so that the friction between the rotating upper part 1 and the lower part 2 is low. It is possible to use metal in the lower part och plastic in the upper part or vice versa. The metal must in that case be non-magnetic.

As examples on suitable dimensions, the plate 4 can be a circular bowl with an outer diameter of 88.5 cm and 6 mm high. The part 5 containing the magnet 6 can be a circular disc 59 mm in diameter and 11 mm high. The lower part 2 can be shaped like a circular bowl with outer diameter of 76 mm, inner diameter of 60 mm, outer height 10 mm and inner height (depth) 8 mm.

### Operation

As mentioned in the introduction, the rotaplater can be used both for enumeration of microorganisms as illustrated in Fig. 4A and for colony isolation as illustrated in Fig. 4B.

### Methodology.

Enumeration. After initial laboratory trials the rotaplater was tested by a five member team in a field trial spanning five weeks and yielding approximately 500 plates per week. It was proposed that two members would use the rotaplater and two would serve as controls, and the operators would alternate as control workers.

Colony isolation. The plates were inoculated in two different ways depending on whether the source of the inoculum was a liquid culture or a colony on an agar plate. A drop of liquid culture, 0,1 ml, was placed close to the centre of the plate and then a bacteriological loop was dragged from the inoculum towards the edge of the plate along the radius. Using the solid sample a drop of sterile buffer was placed close to the centre of the plate. Using a bacteriological loop, a colony from another plate was suspended in the drop of buffer on the plate. The loop was then dragged as described for the liquid culture.

### Results.

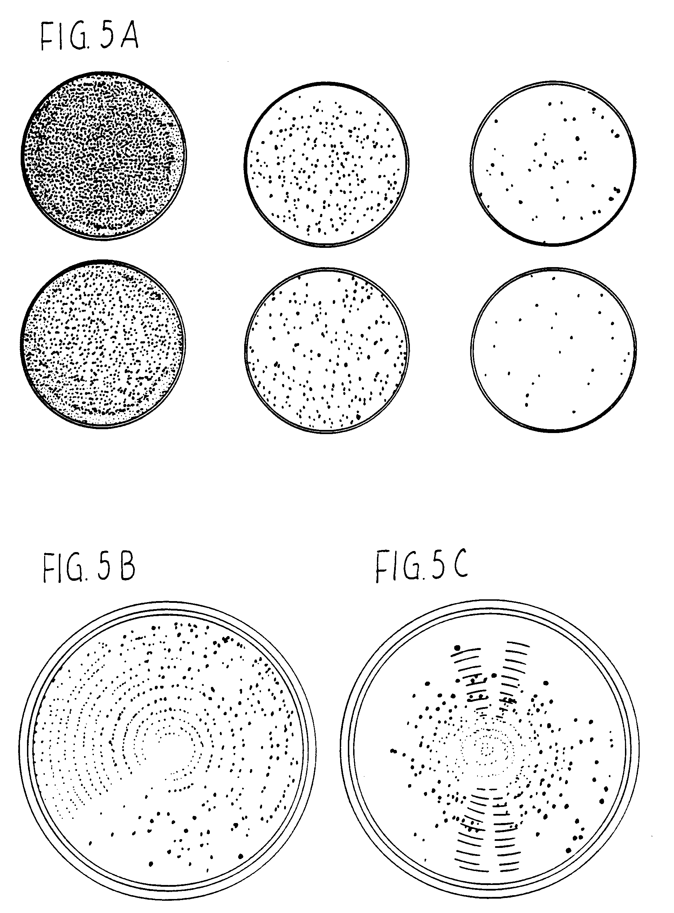

The four persons asked to evaluate the device during their routine laboratory work were all very pleased with the results. All continue to use the device on a regular basis. After the first of the five field trials, all members demanded rotaplaters and results comparing manual and rotaplater operations are only available from this one study. Operator A and operator B reduced the time taken to spread 60 plates by 50% and 40%, respectively. Operator C refused to act as a manual control. Operator D was very recently trained and was unaccustomed to spreading up to 150 plates per day. She suffered discomfort in the hand for several days after spreading all plates manually. Typical results from the field trial are illustrated in Fig. 5A. A uniform distribution of colonies was obtained for all dilutions thus enabling easy enumeration. The laboratory staff concluded that the plates which were spread by the rotaplater were much easier to count than plates from the control group. The results obtained from the colony isolation experiment are illustrated in Fig. 5B and C. The colonies isolated from the liquid culture are illustrated in Fig 5B and the colony shown in Fig 5C were isolated from a solid sample. The separation of colonies on the agar plate was completely acceptable and yielded higher numbers of individual colonies than the conventional streak dilution method.

### Conclusion

It is concluded that this device filled a void for all microbiologists by providing a simple, time saving, inexpensive and easy to operate a apparatus which yields results comparable to or better than manual control results.

### References.

(1) Jarvis, B., Lach, V.H., Wood, J.M. (1977) Evaluation of the spiral plate maker for the enumeration of Micro-organisms in foods. J. Appl. Bact. 43, 149-157.
(2) Trotman, R.E. (1971) The automatic spreading of bacterial cultures over a solid agar plate. J. Appl. Bact. 34, 615-616.
(3) Fraser-Williams, R., Banbury, J.M. (1968) Mechanical rotary device for plating out bacteria on solid medium. J. Clin. Path. 21, 784.

As a further development, the magnet 6 can be replaced with a core of a material that can rotate the upper part 1 in the lower part 2 under influence of a magnetic field. A suitable material is a core of iron instead of the magnet 6. Other material can be used, provided it allows rotation under the influence of a magnetic field.

## Claims

1. A device for spreading microorganisms on a surface, preferably on an agar plate,
**characterised** in that the device consists of an upper part (1) containing a material that can bring the upper part (1) to rotation in the lower part (2) during the influence of a magnetic field, the function of the upper part (1) being to support a plate, preferably an agar plate.

2. A device according to claim 1,
**characterised** in that the device consists of an upper part (1) containing a magnet (6).

3. A device according to claim 1,
**characterised** in that the upper part (1) contains a core made out of iron.

4. A device according to claim 1,
**characterised** in that the upper part (1) consists of a circular plate (4) in the shape of a bowl in which an agar plate can be placed and below plate (4) a circular plate (5) containing a magnet (6) is mounted.

5. A device according to claim 4,
**characterised** in that the upper part (1) is made out of one piece.

6. A device according to claim 1,
**characterised** in that the lower part (2) has the shape of a circular bowl so constructed that it accomodates the plate (5) thereby enabling the plate (5) to rotate in the lower part (2).

7. A device according to claim 1-6,
**characterised** in that the device is made in plastic.

8. A device according to claim 1-6,
**characterised** in that the device is partly made out of metal, preferably in such way that the lower part is made in an non-magnetic metal material and the upper part out of a plastic material or vice versa.

9. A method for the spreading of microorganisms with the use of a device according to claims 1-8,
**characterised** in that a plate, preferably an agar plate is placed on the circular plate (4), and that the upper part of the device (1) is placed in the lower part (2), which is stabilized on a magnetic stirrer so that the upper part (1) of the device is brought to rotation with the help of a magnetic stirrer and the agar plate is inoculated during the rotation.

10. A method according to claim 9,
**characterised** in that during the inoculation of the rotating agar plate an inoculation loop is dragged from the centre of the plate towards the edge of the plate along the radius.

11. A method according to claim 9,
**characterised** in that spreading of microorganisms on the agar plate is done with a spreader in that a small part of the bacterial suspension is placed in the centre of the agar plate and that the spreader first is placed in the centre of the plate and then moved backwards and forwards on the surface of the agar plate.

## Patentansprüche

1. Eine Vorrichtung zum Verteilen von Mikroorganismen auf eine Fläche, vorzugsweise auf eine Agarplatte, dadurch **gekennzeichnet,** daß die Vorrichtung aus einem oberen Teil (1) besteht, das ein Material enthält, das den oberen Teil (1) unter Einfluß eines magnetischen Feldes im unteren Teil (2) zum Rotieren bringen kann, wobei das obere Teil dann die Funktion als Träger einer Platte hat, vorzugsweise eine Agarplatte.

2. Eine Vorrichtung nach Patentanspruch 1, dadurch **gekennzeichnet,** daß das obere Teil (1) einen Magneten (6) enthält.

3. Eine Vorrichtung nach Patentanspruch 1, dadurch **gekennzeichnet,** daß das obere Teil (1) einen Kern aus Eisen enthält.

4. Eine Vorrichtung nach Patentanspruch 1, dadurch **gekennzeichnet,** daß das obere Teil (1) aus einer kreisförmigen Platte (4) in Form einer Schale besteht, in die eine Agarplatte eingelegt werden kann und unter der Platte (4) eine kreisförmige Platte (5) angesetzt ist, die einen Magneten (6) enthält.

5. Eine Vorrichtung nach Patentanspruch 4, dadurch **gekennzeichnet,** daß das obere Teil (1) aus einem Stück gefertigt ist.

6. Eine Vorrichtung nach Patentanspruch 1, dadurch **gekennzeichnet,** daß das untere Teil (2) die Form einer runden Schale hat, die so konstruiert ist, daß sie die Platte (5) aufnehmen kann und dadurch der Platte (5) ermöglicht, im unteren Teil (2) rundzulaufen.

7. Eine Vorrichtung nach den Patentansprüchen 1 - 6, dadurch **gekennzeichnet,** daß die Vorrichtung in Kunststoff ausgebildet ist.

8. Eine Vorrichtung nach den Patentansprüchen 1 - 6, dadurch **gekennzeichnet,** daß die Vorrichtung teilweise in Metall gefertigt ist, vorzugsweise in der Konzeption, daß das untere Teil in einem amagnetischen Material und das obere Teil in Kunststoff - oder umgekehrt - gefertigt sind.

9. Ein Verfahren zum Verteilen von Mikroorganismen unter Benutzung der Vorrichtung nach den Patentansprüchen 1 - 8, dadurch **gekennzeichnet,** daß eine Platte, vorzugsweise eine Agarplatte auf der kreisförmigen Platte (4) plaziert ist, und daß das obere Teil (1) in das untere Teil (2) eingebracht ist, das auf einem magnetischen Rührwerk stabilisiert ist, so daß das obere Teil (1) der Vorrichtungmit Hilfe eines magnetischen Rührwerkes zum Rundlaufen gebracht worden und die Agarplatte während des Rundlaufes beimpft wird.

10. Ein Verfahren nach Patentanspruch 9, dadurch **gekennzeichnet,** daß während der Beimpfung der rundlaufenden Agarplatte eine Beimpfungsschleife von der Plattenmitte entlang dem Radius zum Plattenrand gezogen wird.

11. Ein Verfahren nach Patentanspruch 9, dadurch **gekennzeichnet,** daß das Verteilen der Mikroorganismen auf der Agarplatte mit einem Verteiler erfolgt, indem eine kleine Menge der bakteriellen Suspension in die Mitte der Agarplatte eingegeben wird und der Verteiler zuerst in der Mitte der Platte angeordnet ist, um dann auf der Oberfläche der Agarplatte hin und her bewegt zu werden.

## Revendications

1. Un dispositif pour la dispersion de micro-organismes sur une surface, de préférence sur une plaque gélose, **caractérisé** en ce que le dispositif se compose d'une partie supérieure (1) contenant une matière qui peut amener la partie supérieure (1) en rotation dans la partie inférieure (2) durant l'influence d'un champ magnétique, la fonction de la partie supérieure (1) étant de supporter la plaque, de préférence une plaque gélose.

2. Un dispositif selon la revendication 1, **caractérisé** en ce que le dispositif se compose d'une partie supérieure (1) contenant un aimant (6).

3. Un dispositif selon la revendication 1, **caractérisé** en ce la partie supérieure (1) contient un noyau réalisé de fer.

4. Un dispositif selon la revendication 1, **caractérisé** en ce que la partie supérieure (1) se compose d'une plaque circulaire (4) ayant la forme d'un bol dans laquelle une plaque gélose peut être placée et sous la plaque (4) une plaque circulaire (5) contentant un aimant (6) est montée.

5. Un dispositif selon la revendication 4, **caractérisé** en ce que la partie supérieure (1) est réalisée en un seul bloc.

6. Un dispositif selon la revendication 1, **caractérisé** en ce que la partie inférieure (2) a la forme d'un bol circulaire construit de façon à recevoir la plaque (5) permettant ainsi à la plaque (5) de tourner dans la partie inférieure (2).

7. Un dispositif selon les revendications 1 à 6, **caractérisé** en ce que le dispositif est réalisé de plastique.

8. Un dispositif selon les revendications 1 à 6, **caractérisé** en ce que le dispositif est partiellement réalisé de métal, de préférence de manière que la partie inférieure soit réalisée d'un métal non-magnétique et la partie supérieure d'une matière plastique ou vice versa.

9. Une méthode pour la dispersion de micro-organismes utilisant un dispositif selon les revendications 1 à 8, **caractérisée** en ce que une plaque, de préférence une plaque gélose est placée sur la plaque circulaire (4) et que la partie supérieure du dispositif (1) est placée dans la partie inférieure (2) laquelle est stabilisée sur un agitateur magnétique de manière que la partie supérieure (1) du dispositif soit amenée en rotation au moyen d'un agitateur magnétique et la plaque gélose soit inoculée pendant la rotation.

10. Un dispositif selon la revendication 9, **caractérisé** en ce que pendant l'inoculation de la plaque gélose tournante une boucle d'inoculation est entraînée du centre de la plaque vers le bord de la plaque le long du rayon.

11. Un dispositif selon la revendication 9, **caractérisé** en ce que la dispersion des micro-organismes sur la plaque gélose est effectuée au moyen d'un disperseur de manière qu'une petite partie de la suspension bactérienne soit placée au centre de la plaque gélose et que le disperseur d'abord soit placé au centre de la plaque et ensuite déplacé en arrière et en avant sur la surface de la plaque gélose.
